# EUROPEAN PATENT SPECIFICATION

(11) **EP 2 292 728 B1**
(45) Date of publication and mention of the grant of the patent: **23.03.2016**
(21) Application number: 09773451.1
(22) Date of filing: 30.06.2009
(51) Int. Cl.: C12M 1/00, C12M 1/38, C12M 3/00

(54) **CONSTANT-TEMPERATURE EQUIPMENT**
VORRICHTUNG ZUR TEMPERATURKONSTANTHALTUNG
ÉQUIPEMENT À TEMPÉRATURE CONSTANTE

(30) Priority: 01.07.2008 JP 2008172485
(43) Date of publication of application: 09.03.2011
(73) Proprietor: Rorze Corporation, Hiroshima-ken 720-2104 (JP)
(72) Inventor: YAMASHITA, Seishi, Fukuyama-shi Hiroshima 720-2104 (JP)
(74) Representative: Kramer Barske Schmidtchen Patentanwälte PartG mbB
(86) International application number: PCT/JP2009/061901
(87) International publication number: WO 2010/001873

(56) References cited:
- WO-A1-03/008103
- JP-A- 11 166 555
- JP-A- 2007 132 467
- JP-U- 5 088 298
- JP-U- 5 096 564
- US-A1- 2007 041 814

## Description

### FIELD OF THE INVENTION

The invention relates to constant-temperature equipment for maintaining at least an even temperature.

### BACKGROUND OF THE INVENTION

Constant-temperature equipment has been made use of to culture or test microbes and cells and so on. The constant-temperature equipment is formed by providing with a means for maintaining environmental conditions such as the temperature, the humidity and the density of carbon dioxide to a temperature-controlled chamber for containing many samples which are the subject of culturing and testing. Culturing and testing are carried out continuously for many hours, and during these processes, it is necessary to regularly grasp conditions of each sample. Therefore, it is indispensable to regularly take out the sample from temperature-controlled chamber to examine and analyze. Therefore, many of constant-temperature equipments introduced recently are equipped with memory means arithmetic means and conveyance mechanism to automate their process. The equipment has a function for automatically carrying out putting a container with sample in and out of, delivering it to the processes for examining and analyzing, and handling the sample conditions. According to this, culturing and testing for the long term can be efficiently carried out.

### PRIOR ART

### PATENT LITERATURE

Patent literature 1 : Japanese Patent Laid Open Publication No.

Patent literature 2 : Japanese Patent Laid Open Publication No. 2005-500522

US 2007/041814 A1 relates to storage systems for articles comprising one or more storage modules each having a controlled internal environmental from which said articles may be removed and returned robotically, wherein the individual components of the storage system are fixed to each other.

### DISCLOSURE OF THE INVENTION

### PROBLEMS TO BE SOLVED BY THE INVENTION

However, as for conventional constant-temperature equipment with an automatic conveying function, various problems are caused on carrying out culturing and testing because there are mechanical and electrical components in the temperature-controlled chamber. The conventional constant-temperature equipment is affected by environments such as the temperature and the humidity during culturing to cause high frequency of trouble. Even if repair or maintenance is carried out, much time is required and culturing and testing starts late because its structure is complicated. Besides, when a trouble occurs in the middle of culturing or testing, culturing and testing may be greatly affected on the reliability of themselves.

On the other hand, miscellaneous germs must be removed from the inside of the temperature-controlled chamber before starting culturing and testing. Because new cells or microorganisms to be next cultured and tested are affected and the reliability of culturing and testing for the long time may be harmed in case miscellaneous germs in the air or cells and microorganisms previously used in culturing and testing are left in the temperature-controlled chamber. Accordingly, in the conventional incubator, sterilizing treatments such as ultraviolet sterilization or dry sterilization have been carried out, or sterilization has been carried out by wiping off with medical solution.

However, various problems occur on carrying out the above-mentioned sterilizing treatments with the conventional control-temperature equipment having the automatic conveying function. For example, in the ultraviolet sterilization, ultraviolet rays do not reach the shadow of a structure, and therefore, they must be applied from various directions many times. In case of the dry sterilization, electric components and sealing members are damaged at high temperature from 150 °C to 180 °C. Accordingly, the dry sterilization has been carried out at low temperature which does not damage the components, or the inside has been taken apart to wipe off with the medical solution.

In case of the constant-temperature equipment with a small opening only for automatic conveyance to take the container in and out, even if a door is provided to keep the inside of the chamber isolated from the outside, it must be opened when taking the container in and out. The atmosphere inside the temperature-controlled chamber is affected by the outside environment to decline the reliability of culturing and testing. The culturing and testing are carried out over the long time, and therefore, the above-mentioned problems are strongly hoped for a solution.

The invention is to be thought up to effectively solve the above-mentioned problems.

### MEANS TO SOLVE THE PROBLEM

The constant-temperature equipment of the invention comprises a temperature-controlled chamber with a closed space surrounded by walls, a sample table arranged removably in the temperature-controlled chamber, a sample shelf loaded on the sample table, the sample shelf storing containers containing samples, a plurality of driven magnets fitted on the sample table, a plurality of magnets arranged correspondingly to positions of the sample table having the driven magnets fitted, a housing unit for prescribing the configuration of the magnets, means for generating shifting magnetic field with the magnets implemented on the housing unit, and means for installing the housing unit from the outside of the walls of the temperature-controlled chamber so that the magnets magnetically combine with the driven magnets.

The temperature-controlled chamber of the invention can at least keep the indoor temperature constant, besides being a part of the constant-temperature equipment with a function for regulating the gas density such as moisture, oxygen, nitrogen, carbon dioxide, in which samples being stored. Specially, the constant-temperature equipment of this invention is the most suitable for a culture vessel to be used in biology.

### EFFECTS OF THE INVENTION

There are no electrical and mechanical structures for moving the sample table in the temperature-controlled chamber, and therefore, all structures in it can be dry-heat-sterilized at high temperature. In addition, the sample table can be moved in non-contact through the walls of the temperature-controlled chamber, and therefore, the structure for moving the sample table is not affected by atmosphere having high temperature and high humidity in the temperature-controlled chamber.

If the electrical and mechanical structures should break down during culturing and testing, they can be repaired by removing only the housing unit. Therefore, it is possible to avoid an interruption of culturing and testing. In addition, a means for keeping the humidity during culturing and testing also can be provided to maintain a good environment for culturing.

### BRIEF DESCRIPTION OF THE DRAWINGS

Fig. 1 is a view for explaining constant-temperature equipment of an embodiment of the invention.
Fig. 2 is a view of a removable part of constant-temperature equipment of an embodiment of the invention.
Fig. 3 is a section view of constant-temperature equipment of an embodiment of the invention.
Fig. 4 is a section view for explaining an embodiment of conveyance means.
Fig. 5 is a view for explaining a physical relationship between conveyance means and a conveyed article.
Fig. 6 is a view for explaining the proper procedure for conveying a conveyed article with conveyance means.
Fig. 7 is a view for explaining engaging means of shielding means.
Fig. 8 is a view for explaining a cross section of a shielding plate.
Fig. 9 is a view for explaining a form of magnets arranged on sample table drive means.
Fig. 10 is a view for explaining a form of magnets arranged on a sample table.
Fig. 11 is a view for explaining an example of conveyance means.
Fig. 12 is a view for explaining another example of conveyance means.
Fig. 13 is a view for explaining a water saving portion in an example, which is provided on a floor of a temperature-controlled chamber.
Fig. 14 is a view for explaining a configuration of magnets.
Fig. 15 is a view for explaining a drive means using electro-magnets.

### EXPLANATION OF REFERENCED NUMERALS

- 1: constant-temperature equipment
- 2: a box
- 3: a door
- 4: a sample shelf
- 5: a sample table
- 6: sample table drive means
- 7: a large opening
- 8: a small opening
- 9: a shielding plate
- 10: a slide frame
- 11: conveyance means
- 12: travel means
- 13: an external wall for temperature-controlled chamber
- 14: a sample container
- 15: a temperature-controlled chamber
- 16: an internal wall
- 17: a space
- 18: a base board
- 19: a rolling body
- 20: a locational tool
- 21: a driven magnet
- 22: heat insulating material
- 23: a housing unit
- 24: a gear motor
- 25 (25a, 25b): a pulley
- 26: a belt
- 27: a magnetic housing
- 28: a spacer
- 29: a bearing
- 30: a driving magnet
- 31: a circular table
- 32: a shield
- 33: a finger
- 34: a spacer
- 35: a slewing table
- 36: a gear motor
- 37a: a first arm
- 37b: a second arm
- 38: a bearing
- 39: a base shelf
- 40: a plate
- 41: a gear
- 42: a slewing motor
- 43: a gear
- 44: a shield shelf
- 45: a fixing block
- 46: a block
- 47: a top plate
- 48: an engaging pin
- 49: a sensor
- 50: a opening/closing door
- 51: a rotary source
- 52: a side wall
- 53: a pass box
- 54: an elevator gear motor
- 55: a pinion gear
- 56: a slide guide
- 57: a move terminal
- 58: an elevator base
- 59: a rack gear
- 60: a rack base
- 61: a solenoid
- 62: a micro-opening
- 63: a bracket
- 64: a bracket
- 65: an upper shielding plate
- 66: a long hole
- 67: a slewing shielding plate
- 68: a fixed shielding plate
- 69: a pulley
- 70: a screw hole
- 71: a shielding plate
- 72: an extract hole
- 73: a bearing
- 74: a pulley
- 75: a belt
- 76: an engaging hole
- 77: an engaging unit
- 78: a bracket
- 79: a water saving portion
- 80: an electro-magnet unit

### PREFERRED EMBODIMENT OF THE INVENTION

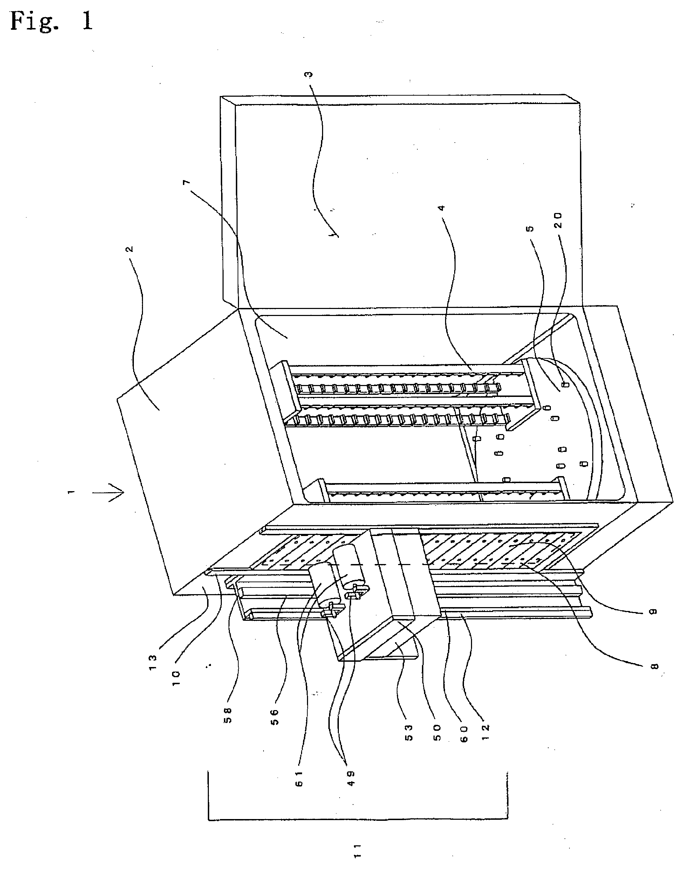

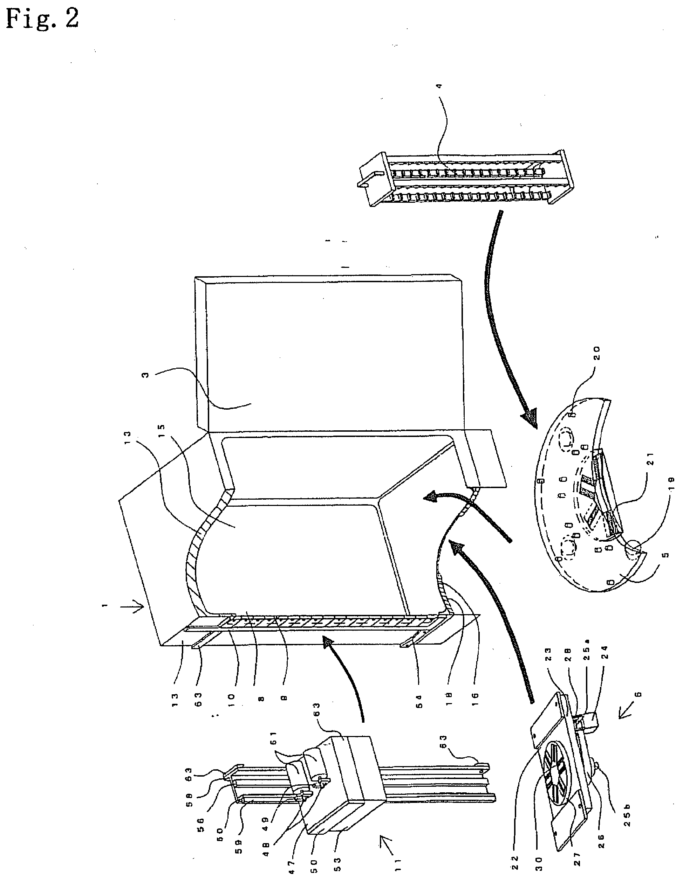

An embodiment of the invention will be explained with reference to following drawings. Fig. 1 is a perspective view of constant-temperature equipment 1 of this embodiment, and Fig. 2 illustrates a state for separating parts removable from the constant-temperature equipment 1. The constant-temperature equipment 1 comprises a box 2, a door 3, a sample table 5 for placing a sample shelf 4, and sample table drive means 6 for rotating the sample table 5. The box 2 has two openings. One is a large opening 7 for taking the sample shelf 4 or the sample table 5 in and out, which is used also to carry out maintenance. The other is a small opening 8 for taking a sample container 14 that is loaded on each shelf of the sample shelf 4. The small opening 8 forms a liner opening having a length for exposing all shelves of the sample shelf 4 to the outside of the constant-temperature equipment 1. The door 3, which blocks the opening, is provided to the large opening 7 through hinges so as to be freely opened and closed.

A plurality of shielding plates 9 are vertically piled on the small opening 8 movably along a slide frame 10 in accordance with the number of shelves in the vertical direction of the sample shelf 4. Each shielding plate 9 has an area enough to individually take the sample container 14 in and out. The slide frame 10 guides the shielding plates 9 in the vertical direction. Here, the temperature-controlled chamber 15 forms a space isolated from the outside by shielding the small opening 8 with the shielding plates 9.

Conveyance means-11 having a function for taking the sample container 14 in and out is detachably provided to the position facing the shielding plates 9. In addition, the conveyance means 11 is gone up and down by travel means 12 which is a part of the conveyance means 11. According to this, combinations of various workings of the drive means provided to the conveyance means 11 have the shielding plate 9 opened and closed, and the sample container 14 held up, brought in and out, and placed. Furthermore, the conveyance means 11 is detachably installed on an external wall 13 of the constant-temperature equipment 1, which can be removed from the constant-temperature equipment 1.

Means for keeping an environment in the temperature-controlled chamber 15, which is not illustrated in drawings, is provided in a space 17 formed by an internal wall 16 of the temperature-controlled chamber 15 and the external wall 13. Environment conditions such as the temperature inside the temperature-controlled chamber 15 and the density of carbon dioxide therein are kept in required conditions.

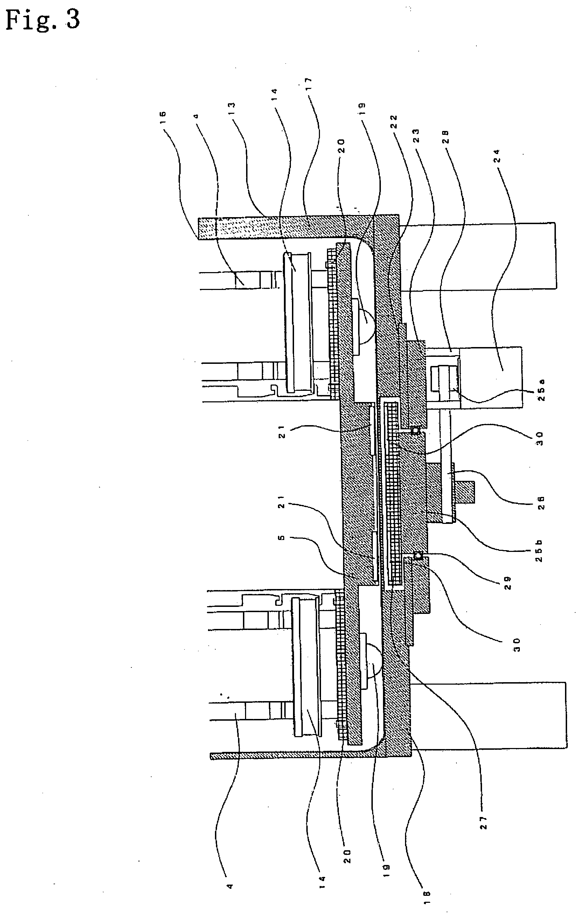

Next, internal structure of the box 2 and the sample table drive means 6 will be explained with reference to Fig. 2 and Fig. 3. The internal wall 16 and the external wall 13 are fixed on a base board 18. The internal wall 16 has a function for perfectly blocking except for the large opening 7 and the small opening 8. The temperature-controlled chamber 15 contains the sample container 14 including a sample, the sample shelf 4 for putting a plurality of sample containers 14, and the sample table 5 for placing a plurality of sample shelves 4.

The sample table 5 has rolling bodies 19 such as a ball caster fitted on the back side of the bottom at regular intervals so as to be installed movably on the floor inside the temperature-controlled chamber 15. In addition, the sample table 5 has driven magnets 21 fixedly placed on the back side so as to follow the working of driving magnets 30 which are provided to the sample table drive means 6 as a magnetic generator. Although this invention is subject to the rolling bodies 19, even in another example wherein an assembly made with materials having low frictional resistance instead of a rolling member is fitted on the bottom, the same effects can be obtained. Moreover, the sample table 5 has a locational tool 20 provided on the top, enabling to position in placing the sample shelf 4.

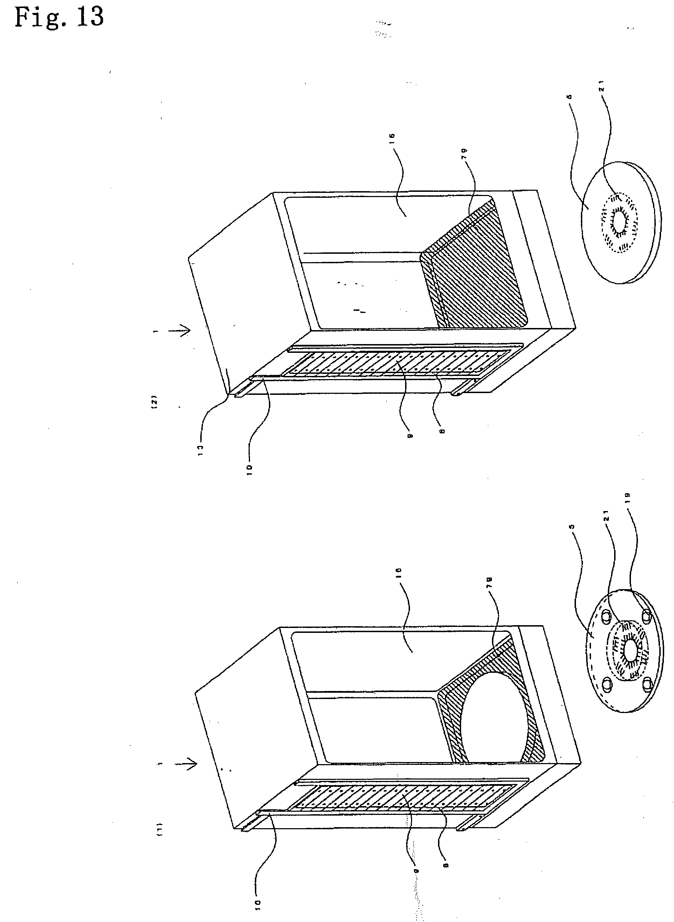

In examples shown in Figs. 13(1) and 13(2), a water saving portion 79 for keeping the humidity is provided therein. In Fig. 13(1), a cavity for saving demineralized water is formed by lowering the floor on which the rolling body 19 works. In addition, the temperature-controlled chamber 15 saves demineralized water throughout the bottom. In the example of Fig. 13(2), the temperature-controlled chamber 15 has a function for keeping the humidity by filling the bottom with the demineralized water. In this case, the water resisting property is improved by using the rolling body 19 made of resin or by covering all the bottom of the sample table 5 with low frictional resistance member.

The sample table drive means 6, which drives the sample shelf 4 in optional direction and at optional speed, is installed on the back side of the bottom of a base board 18 through heat insulating material 22. According to this, even the inside of the temperature-controlled chamber 15 is kept at high temperature, components for constituting the sample table drive means 6 are hardly affected by the heat in the temperature-controlled chamber 15. The sample table drive means 6 comprises a housing unit 23 like a plate, a gear motor 24, a pulley 25a, 25b, a belt 26 and a magnetic housing 27. The gear motor 24 is installed on the housing unit 23 through a spacer 28. The sample table drive means 6 has all of the components implemented on the housing unit 23, being attached removably to the temperature-controlled chamber 15 as one body by fitting the housing unit 23 to the outside of the base board 18 with screws. The housing unit 23 can be also fixed with bolts. In case a screw with a hand grip, it is possible to easily carry out maintenances.

The gear motor 24 is electrically connected to a not-illustrated control assembly to set up parameters of the workings by input means like a keyboard. The pulley 25a is fixed to the axis of rotation of the gear motor 24. The rotary force of the gear motor 24 is transmitted to the pulley 25b by the belt 26. Here, the pulley 25b is rotatitively assembled to the housing unit 23 through bearings 29.

The pulley 25b has the magnetic housing 27 fixing a plurality of driving magnets 30 installed. Rotations of the motor are transmitted to the magnetic housing 27 at a fixed speed ratio through the pulleys 25a, 25b and the belt 26 to generate shifting magnetic field. Besides, the driving magnets 30 are fixed to the magnetic poles of the driven magnets 21 fixed on the bottom of the sample table 5 in a combination so that they pull against each other. According to this, the workings of the magnetic housing 27 are transmitted to the sample table 5 through the magnetic force to operate the sample table 5. In this case, the base board 18 and the walls of the temperature-controlled chamber 15A are formed by materials having no magnetism or poor magnetism. In addition, according to this structure, when bringing out the sample table 5 from the temperature-controlled chamber 15 to the outside, it can be brought out by merely picking up with one hand without tools.

There is no structure on the bottom of the temperature-controlled chamber 15. The positioning member is not also provided. Therefore, means for positioning in placing the sample table 5 becomes a problem. However, the sample table 5 can be located at the almost same position with before it is detached because it is introduced to the sample table drive means 6 by mutual magnetic force of the magnets arranged on the magnetic housing 27 of the sample table 5 and the sample table drive means 6. Besides, a self-aligning action works because the sample table 5 rotates by following rotary workings of the magnetic housing 27. That is, the sample table 5 can be reinstalled as keeping high positional repeatability by being lead so that the rotational center axis passes along the same line with a rotational center axis of the magnetic housing 27.

The sample table drive means 6 uses a stepping motor or a servo motor as driving force. Accordingly, when the electric current is cut off or the gear motor 24 loses synchronism, positional data have been disappeared occasionally. Therefore, in this case, the position must be confirmed once more. The control assembly can catch the sample table 5 by providing means for detecting positions to the outside of the temperature-controlled chamber 15 and a detected substance to the sample table 5 as means for confirming positions.

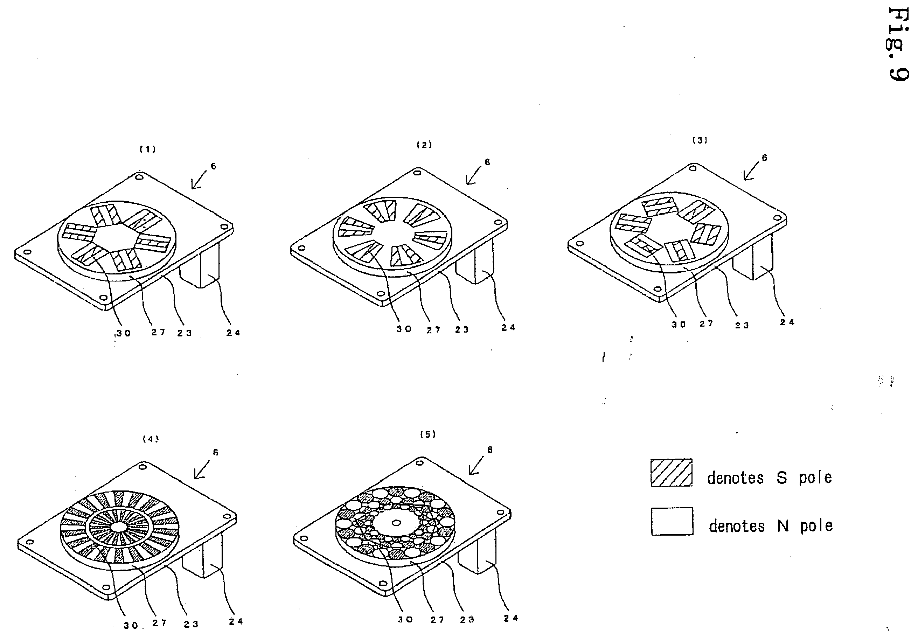

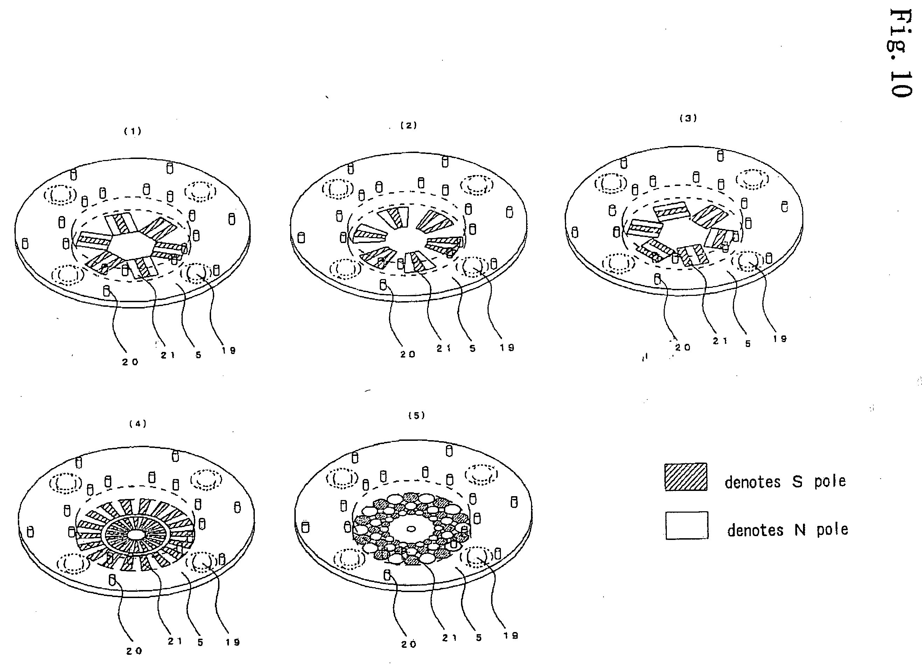

Next, the driven magnets 21 and the driving magnets 30 will be explained. Fig. 9 is a perspective view of the sample table drive means 6. Fig. 10 is a perspective view of the sample table 5, illustrating by peering through the top of the sample table. Both same numbers of Figs. 9(1) to 9(5) and Figs. 10(1) to 10(5) form a combination of driving force transmission. In the example of Figs. 9(1) and 10(1), rectangular magnets are used by combing magnets of three one set for alternately arranging N pole and S pole. There are two kinds of N-S-N and S-N-S as an arrangement of three magnets. In the example, all magnets of the sample table 5 and the magnetic housing 27 are arranged so as to pull against each other only when the sample table 5 rotates at a fixed angle to the magnetic housing 27.

According to this, even if the sample table 5 is removed from the temperature-controlled chamber 15 and reinstalled therein by hand, it can be replaced at the previous rotary position by rotating it therein and locating at the rotary position having the strongest magnetic force.

In Figs. 9(2) and 10(2), magnets of three one set as same as Figs. 9(1) and 10(1) form in a sector from the center of the magnetic housing 27. In Figs. 9(3) and 10(3), the sample table 5 is arranged so that the rotational center is close to the rotational center of the magnet housing 27 by dividing magnets of three one set in the rotational direction and the radial direction. Due to the arrangement of three one set magnetic, power transmission in rotational direction can be effectively obtained. That is, a self-aligning effect can be obtained more effectively.

In Figs. 9(4) and 10(4), stronger magnetic force can be obtained by installing magnets shaped in a sector on the whole magnetic housing 27. In Figs. 9(5) and 10(5), stronger magnetic force can be obtained by installing large and small circular magnets on the whole magnetic housing 27. The magnets have the form and the combination selected in various forms, numbers and arrangements. However, it goes without saying that it is desirable that the driven magnet 21 of the back side of the sample table 5 and the driving magnet 30 of the top of the magnetic housing 27 have common form, number and arrangement and besides the facing magnets have magnetic poles to pull against each other. Further, the driving magnet 30 has the magnetic force stronger by using a yoke.

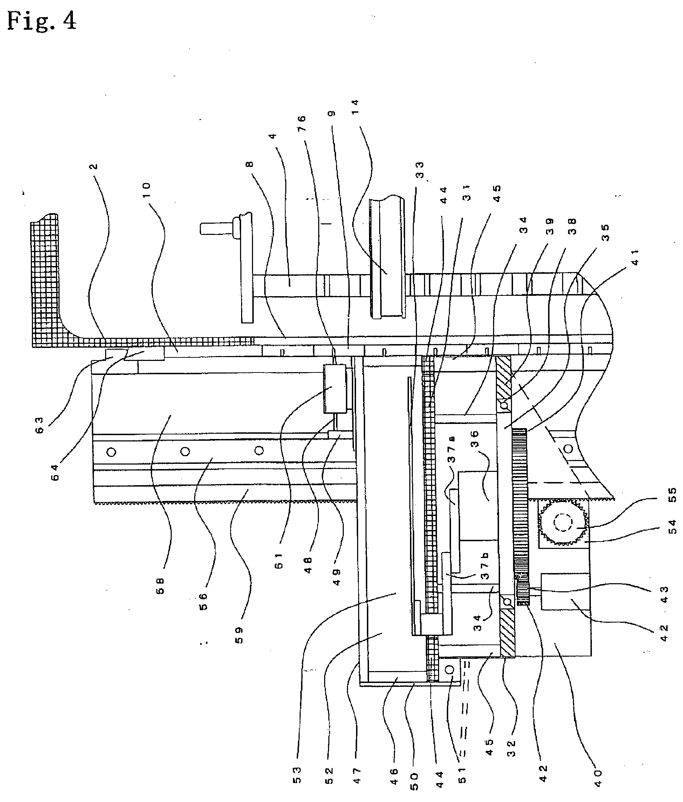

Next, the conveyance means 11 will be explained with reference to Fig. 4. A gear motor 36 is fixed on a slewing table 35, and one end of a first arm 37a is installed to a rotational axis of the gear motor 36. The other end of the first arm 37a is combined to the base end of a second arm 37b, and the other end of the second arm 37b is combined to the base end of a finger 33. Here, the first arm 37a and the second arm 37b constitute an arm mechanism for advancing and backing the finger 33. The gear motor 36 has rotary motions transmitted to advance and back the finger 33 at a fixed speed ratio through the first arm 37a and the second arm 37b by installing them with a fixed speed ratio.

The slewing table 35 and a circular table 31 are fixed with spacers 34. The slewing table 35 is rotatively installed on a base shelf 39 through bearings 38 to form slewing mechanism. The base shelf 39 is fixed on a plate 40. The slewing table 35 is provided with a gear 41 for engaging with a gear 43 which is fixedly fitted to an axis of a slewing motor 42 which is fixed on the plate 40. According to this, the circular table 31 fixed on the slewing table 35, the gear motor 36, and the first arm 37a, the second arm 37b and the finger 33 fitted to the gear motor 36 are rotated by driving the slewing motor 42.

A shield shelf 44, which is provided with an opening having a diameter larger than the circular table 31, is fixed on the base shelf 39 through a fixing block 45 so as to be opposite the circular table 31. Accordingly, the circular table 31 and the shield shelf 44 serve as a partition between a pass box 53 for passing through the sample container 14 and the outside having the driving means.

The shield shelf 44 has a top plate 47 installed on the upper through a block 43. The top plate 47 has a detachable piece (an engaging pin 48 in the example) installed on the upper. The detachable piece is engaged with a concavity which is formed on the plane of the shielding plate 9, and advanced and backed by electromagnetic force of a solenoid 61. The engaging pin 48 pierces through the solenoid 61. When the engaging pin 48 is engaged with the shielding plate 9, it is advanced by the solenoid 61. When the engagement is released, the engaging pin 48 is backed by the solenoid 61. In addition, the engaging pin 48 has a sensor 49 provided to the rear to grasp the position for being advanced and backed.

The shield shelf 44 has an opening/closing door 50 fitted behind the finger 33 so as to freely open and close. The opening/closing door 50 is opened and closed by rotary source 51. Although the solenoid is used as the rotary source 51, a spring, an air cylinder, or a motor can be also used. The shield shelf 44 and the top plate 47 are opposite the shielding plate 9 in a slight opening, having side walls 51 to the left and right sides. Accordingly, when the small opening 8 is opened, the pass box 53 serves as a buffer room for loosening a change of the atmosphere in the temperature-controlled chamber 15. Here, the pass box 53 comprises the shielding plates 9, the top plate 47, the opening/closing door 50, the side walls 52, the slewing table 35, the base shelf 39 and the shield 32. To heighten the isolating property, each contact portion has a not-illustrated rubber sealing member provided.

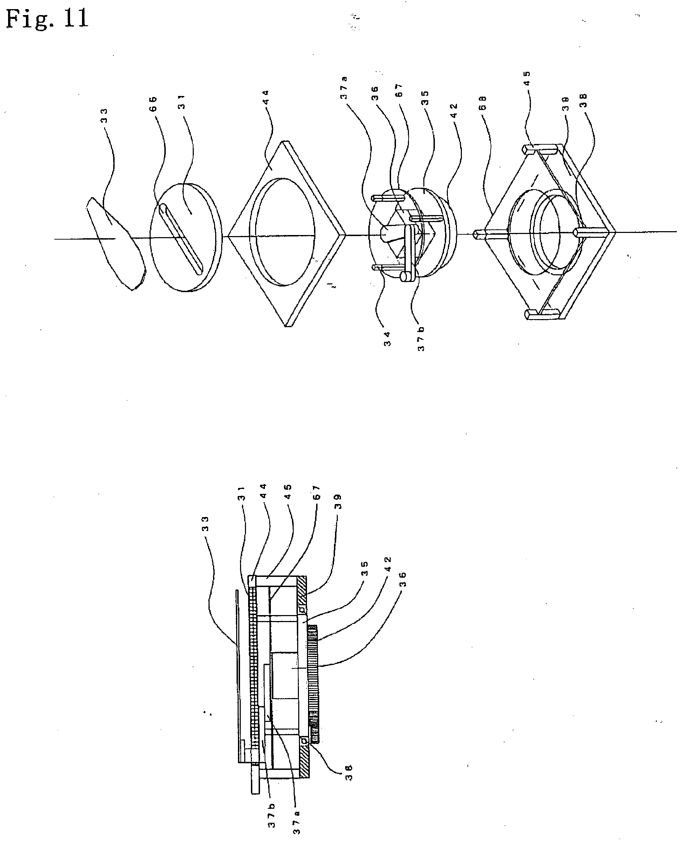

In the above-mentioned example, the circular table 31 has a long hole 66 along working of the finger 33 so that a coupling portion of the second arm 37b and the finger 33 can be operated. However, the motor 36 may be broken by flowing the atmosphere having high temperature and high humidity in the temperature-controlled chamber 15 through the long hole 66 at the time when the shielding plates 9 open. In this case, as shown in Fig. 11 of the other example, the atmosphere can be prevented from entering the motor 36 by providing a slewing shielding plate 67 and a fixed shielding plate 38 between the motor 36 and the first arm 37a.

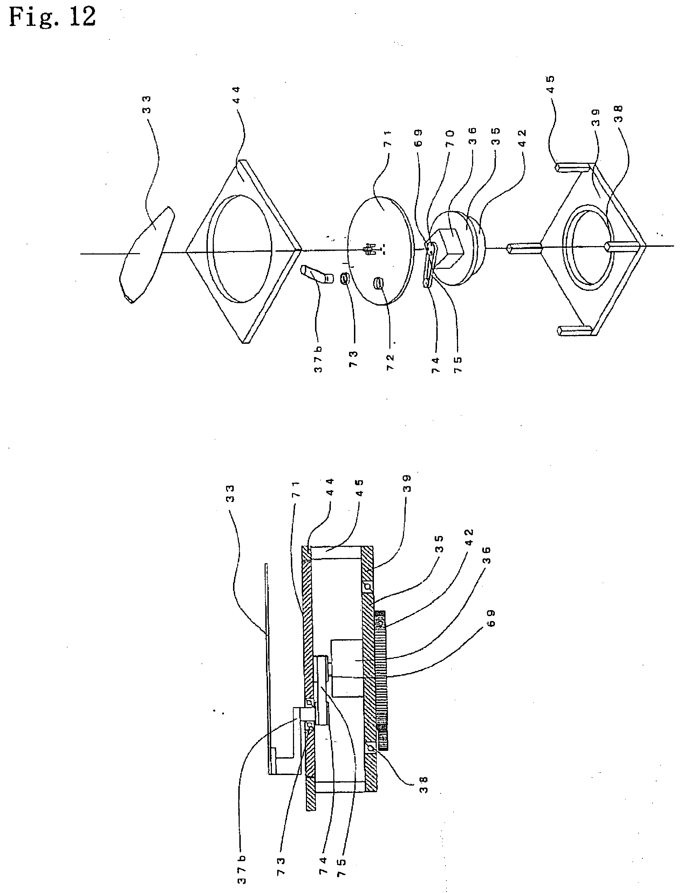

Furthermore, there is another example as shown in Fig. 12. Instead of the first arm 37a, a pulley 69 and a shielding plate 71 are concentrically fixed on the rotational axis of the motor 36. The shielding plate 71 has an extract hole 72 whose center is on the slewing track of the first arm 37a, and therein, the second arm 37b is inserted from the side of the finger 33 through a bearing 73. A pulley 75 is installed to connect the second arm 37b and the first arm 37a. When the rotary speed ratio of the pulley 69 and the pulley 74 is made same as the first arm 37a, the pulley 69 and the shielding plate 71 are rotated by the motor 36. In addition, rotations of the pulley 69 are transmitted to the pulley 74 through a belt 75 to advance and back the second arm 37b through the finger 33.

According to this method, the shield shelf 44 and the shielding plate 71 serve as a partition so that the mechanical structure below the second arm 37b can be isolated from the atmosphere having high temperature and high humidity in the temperature- controlled chamber 15. The isolating property is heightened by providing a bearing between the shielding plate 71 and the shield shelf 44.

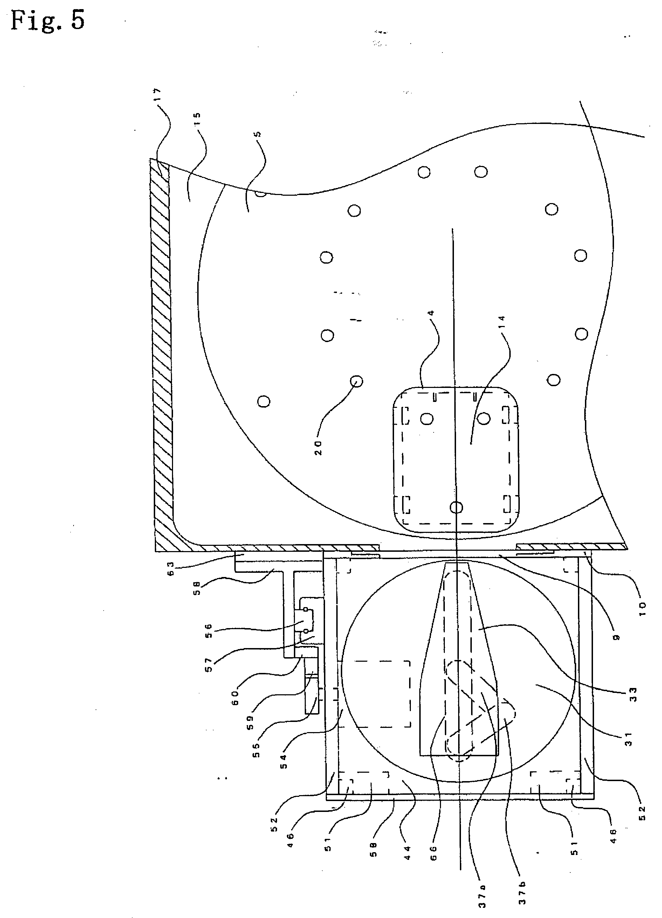

Next, the traveling means 12, which is a part of the conveyance means 11, will be explained with reference to Figs. 4 and 5. The plate 40 has an elevator gear motor 54 for traveling installed, and the motor axis has a pinion gear 55 fixed. In addition, the plate 40 has a move terminal 57 of a slide guide 56 fixed on the back side, moving along the track of the slide guide 56 installed on an elevator base 58. The slide guide 56 of the elevator base 58 has a rack gear 59 having teeth for engaging with the pinion gear 55 installed through a rack base 60.

The elevator base 58 and the rack base 60 are detachably installed to the box 2 through brackets 63, 64 so that the engaging pins more than one engage in engaging holes 76 of the opposite shielding plates 9. Therefore, the engaging pins 48 are certainly engaged to the shielding plates 9 which are located at any position from the lowermost shelf to the uppermost shelf. Needless to say, the slide guide 56 and the rack gear 59 have a stroke enough to hold up one step for the shielding plates 9 positioning at every point from the lowermost shelf to the uppermost shelf. Although a rack-and-pinion method is adopted for elevating means of this example, in another example is provided elevating means according to a ball screw, and in further another example is provided elevating means according to a linear servo.

As shown in Fig. 5 wherein the equipment is viewed from the top, at a home position, the shielding plate 9 crosses at right angle to the center line of the finger 33, and the sample table 5 is positioned so that the rotary center passes on a line for extending the center line of the finger 33. The finger 33 is advanced in the temperature-controlled chamber 15 through the small opening 8 by the gear motor 36, stopping at the lower tip position of the sample container 14 put on the sample shelf 4, slightly moving upwardly by rotating the rack gear 59, loading with the sample container 14, thereafter being backed by the gear motor 36. All input-output circuits of the gear motor 36, the slewing motor 42, an elevator gear motor 54, the solenoid 61 and the sensor 49 are electrically connected to a not-illustrated control assembly. An operator can optionally set up the operation with input means such as a key board and so on.

Accordingly, it is possible to bring out the sample container 14 put on the sample shelf 4 in the temperature-controlled chamber 15 or put the sample container 14 on the sample shelf 4 in the temperature-controlled chamber 15 from the outside automatically as the moving sequence set by the operator. The conveyance means 11 can be attached removably from the box 2 as a boundary of the brackets 63, 64. In carrying out the dry sterilization in the temperature-controlled chamber 15, the conveyance means 11 are taken out to avoid the effects of heat of the dry sterilization. According to this, the source of the trouble is decreased. Even if it is out of order, the maintenance can be carried out without affecting the inside of the temperature-controlled chamber 15. Therefore, it is unnecessary to break off culturing and testing expressly and it is possible to carry out efficient culturing and testing.

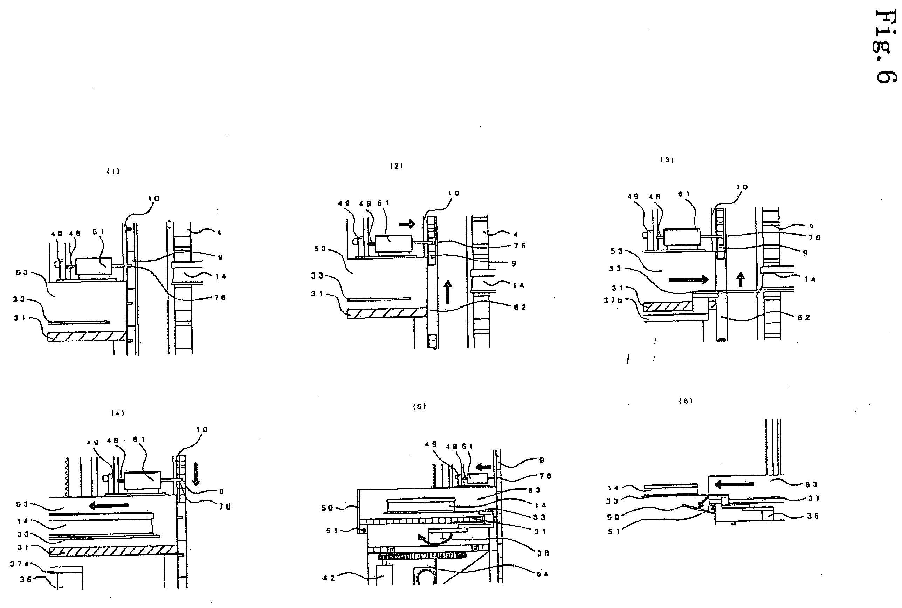

Next, a motion for carrying the sample container 14 of the conveyance means 11 will be explained with reference to Figs. 6(1) to 6(6). Here, black arrows in the figures show a motion direction of each driving portion.

(1) As instructed by the operator, the sample shelf 4 for putting the sample container 14 is rotatively moved to the position where the finger 33 can be carried by the sample table drive means 6 to be stopped. The engaging pin 48 of the conveyance means 11 is elevated to the height of the engaging hole 76 of the shielding plates 9 corresponding to the indicated shelf of the sample shelf 4 by the travel means 12.

(2) Positional data of each motor are sent to the control assembly at any time. When the data reach the appointed position, a signal is sent from the control assembly, so that the solenoid 61 can move the engaging piri 48 toward the shielding plates 9. The control assembly judges according to on-off signals of the sensor 49 provided behind the engaging pin 48 if the engaging pin 48 fully engages in the engaging hole 76. It is possible to confirm the position of the engaging pin 48 by setting so that an optical axis of the sensor 49 can pass between the position where the rear end of the engaging pin 48 engages with the engaging hole 76 and the position insufficient for the engagement.

If the engaging pin 48 insufficiently shades, in other words, if the optical axis of the sensor 49 is kept shaded, it should be set up so that next motion is not carried out. When the engaging pin 48 is sufficiently engaged in the engaging hole 76, the control assembly orders so that the travel means 12 has the conveyance means 11, the shielding plate 9 engaged with the engaging pin 48, and all shielding plates pilled up on the upper from the shielding plates 9 moving in a rising direction by an optional moving amount. According to this, upper shielding plates 65 over the shielding plate 9 engaged with the engaging pin 48 are moved in the rising direction within the slide frame 10 to produce a fine opening 62.

The moving amount is enough to produce the smallest opening possible to bring in and out the sample container 14, being one shelf of the sample shelf 4 for the maximum. According to this motion, the temperature-controlled chamber 15 is opened. However, since an opening isolated with the pass box 53 in the conveyance means 11 occupies only the minimum volume necessary for conveying the sample container 14, the environmental change is held down to a minimum.

(3) When the elevator gear motor 54 finishes raising a shielding plate, the control assembly orders the gear motor 36 to advance the finger to an appointed position. Vertical positions of the finger 33 and the engaging pin 48 and the moving amount for opening the shielding plates 9 are determined according to the height of the sample container 14. When the finder 33 finishes forwardly moving to an appointed position below the sample container 14, the control assembly orders the travel means 12 to slightly rise to hold up the sample container 14.

(4) When the rise by the elevator gear motor 54 is finished and the finger 33 holds up the sample container 14, the control assembly orders the - gear motor 36 to back the finger 33 to the position where it was and the sample container 14 is conveyed from the temperature-controlled chamber 15 to the pass box 53. When the finger 33 is backed to the position where it was and the sample container 14 is conveyed into the pass box 53, the control assembly orders the travel means 12 to lower the conveyance means 11 to the position (1). According to this, the fine opening 62 is shielded and the temperature-controlled chamber 15 is formed in a closed space again.

(5) When the travel means 12 finishes moving the conveyance means 11 downwardly, the control assembly orders the solenoid 61 to back the engaging pin 48 from the engaged state to the position (1). When the sensor 49 confirms that the backing working is finished, the control assembly orders the travel means 12 to go up or go down the conveyance means 11 to the not-illustrated predetermined position of delivery of the sample container 14-After finishing moving to the position of delivery, the control assembly orders the slewing motor 42 to rotate a rotary portion in the opposite direction of 180 degrees.

(6) After the slewing working is finished, the control assembly orders the rotary source 51 of the opening/closing door 50 to open the door. After receiving a signal that opening the door is finished, the control assembly orders the gear motor 36 to advance the finger 33 to an appointed position of delivery. When the finger 33 advances to the position of delivery, the control assembly orders the elevator gear motor 54 to slightly lower the finger 33 on a not-illustrated table and to load the sample container 14 thereon. After the delivery is finished, the control assembly orders the gear motor 36 to put the finger 33 back where it was. After the working is finished, the control assembly orders the gear motor 36 to close the opening/closing door 50. According to this, the pass box 53 again forms a closed space, and then a series of conveyance working is complete.

According to this opening working, the pass box 53 is open to the outside. However, since the volume of the pass box 53 is minimized as much as possible, it is possible to minimize atmosphere replacement with the outside. That is, leakage to the outside of bacteria which floats in the temperature-controlled chamber 15 can be minimized, and inflow of miscellaneous germs from the outside into the temperature-controlled chamber 15 can be also minimized.

As the above-mentioned, the sample container,14 is carried out in order. In carrying in it, each shelf from (1) to (6) can be performed on the contrary.

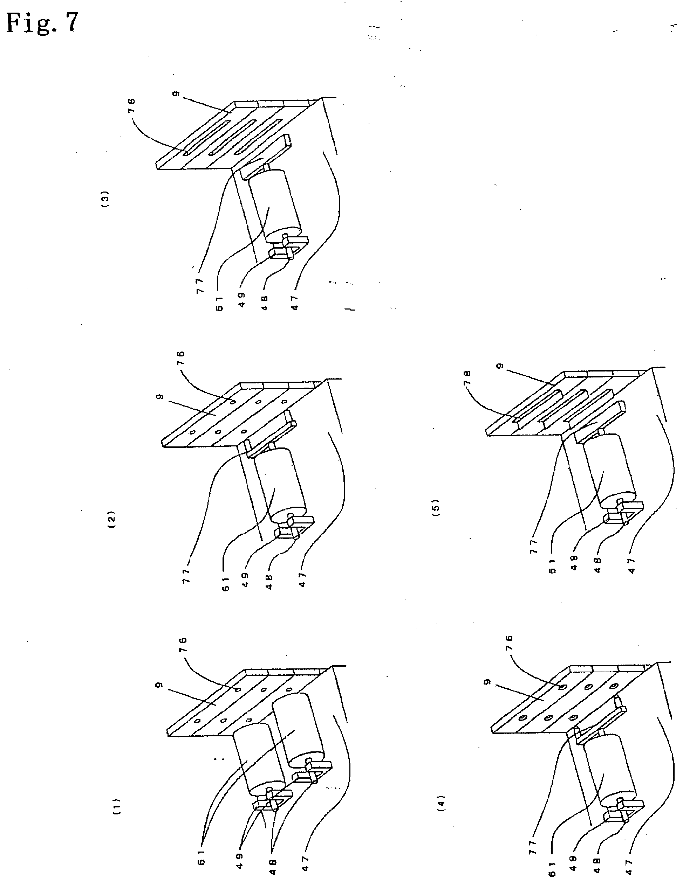

As to engage in an engaging hole 76 which is provided to the shielding plates 9, five kinds of forms as shown in Figs. 7(1) to 7(5) were carried out. In this example of Fig. 7(1), the engaging hole 76 has two engaging pins 49, two solenoids 61 and two sensors 49 provided. In Fig. 7(2), the engaging pin 48 has an engaging unit 77 provided to the tip and the solenoid 61 drives with one. In Fig. 7(3), the engaging hole 76 and the engaging unit 77 are respectively shaped rectangular, thereby enabling to reduce the number of components in comparison with the case in Fig. 7(1). In Fig. 7(4), the engaging hole 76 is shaped tapered, thereby enabling to assure the engagement still more. In Fig. 7(5), instead of the engaging hole 76, a bracket 78 is provided, thereby enabling more assured engagement in comparison with the cases in Figs. 7(1) to 7(4). Although the position for engaging the engaging pin 49 and the engaging hole 76 of each shielding plate 9 is determined by a method for inputting instructing data to the control assembly, the position of the engaging hole 76 can be detected by installing a sensor.

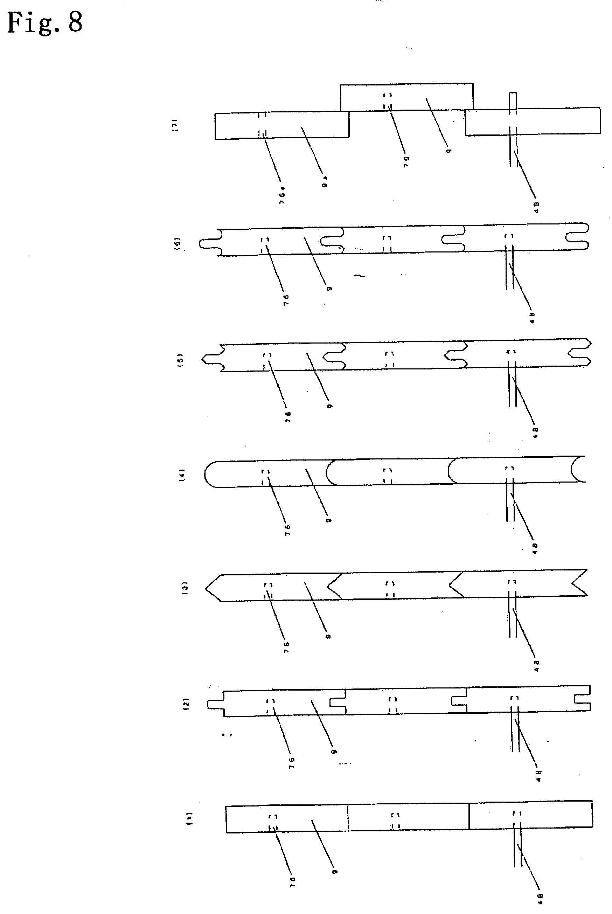

Next, sectional form of the shielding plate will be explained with reference to Figs. 8(1) to 8(7). Fig. 8(1) illustrates a section of the shielding plate 9 of this example, whose form is easy to machine most. However, each section in Figs. 8(2) to 8(7) is adopted to enhance the airtightness. In Fig. 8(2), making unevenness for the top and the bottom of the shielding plate 9 enables to increase contact area between both shielding plates 9 to enhance the airtightness. In Figs. 8(3) and 8(4), the repeat accuracy in opening and closing is enhanced by inclining the interfitted portion. In case of Fig. 8(5) and Fig. 8(6), the airtightness related to Fig. 8(2) and the repeat accuracy each related to Fig. 8(3) and Fig. 8(4) are compatibly enhanced, wherein the uneven portions are inclined or rounded to be easily interfitted.

In an example of Fig. 8(7), the shielding plates 9 are arranged in two rows, whose parts contact with different shielding plates 9 one another. The engaging hole 76a of the shielding plate 9a, which is in front of the engaging pin 48, forms a through hole. The engaging pin 48 has a stroke enough to reach the engaging hole 76 of the shielding plate 9 which is arranged backward. According to this structure, open area equal to Figs. 8(1) to 8(7) can be gained by moving more few shielding plates. In addition, the shielding plates 9 are made members which are attracted by magnetic force in enhancing the airtightness in the examples of Figs. 8(1) to 8(7). During closing, the shielding plates 9 are adsorbed by the contacting slid frame 10 with electromagnets. In opening the small opening 8, the electromagnets have the electricity intercepted.

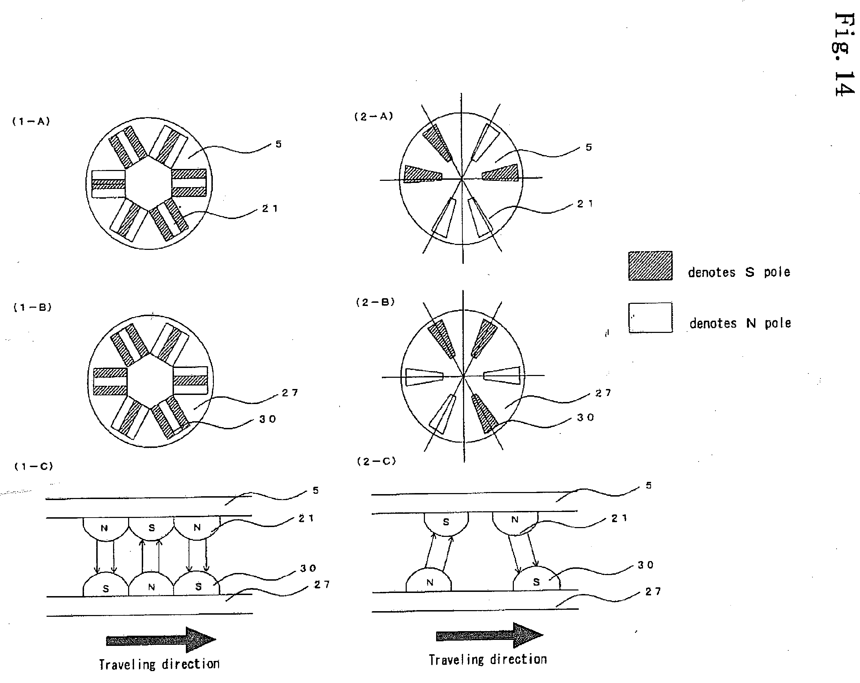

The driven magnets 21 on the back of the table 5 and the driving magnets 30 arranged on the magnet housing 27 will be explained with reference to Fig. 14. Each of Figs. 14(1-A) to 14(1-C) shows the arrangement of the magnets in this example, Fig. 14(1-A) is a view of the sample table 5 seen from the back side, and Fig. 14(1-B) is a view of the magnet housing 27 seen from the top side. Fig. 14(1-C) illustrates a vertical section of the magnets facing each other. In a stationary state, the opposite magnets pull against each other. When the magnet housing 27 is moved in a moving direction, the sample table 5 is moved by two forces which are the pulling force between the opposite magnets and the repulsion between adjacent magnets having the same magnetic polarity.

There are another example of Figs. 14(2-A), 14(2-B) and 14(2-C). Fig. 14(2-A) is a view of the sample table 5 seen from the back side, and Fig. - 14(2-B) is a view of the magnet housing 27 seen from the top side. In Fig. 14(2-B), six driving magnets 30 are arranged on the magnet housing 27 at the same configuration, and the opposite driven magnets 21 are arranged on the sample table 5 so as to be equally shifted to the configuration of the driving magnet 30. Fig. 14(2-C) illustrates a vertical section of the opposite magnets. Since the upper and lower magnets are arranged at the positions having equal magnetic force at a fixed interval without straightly facing, the magnetic force decreases in dispersion. Therefore, it is possible to obtain the equal magnetic force with a few magnets.

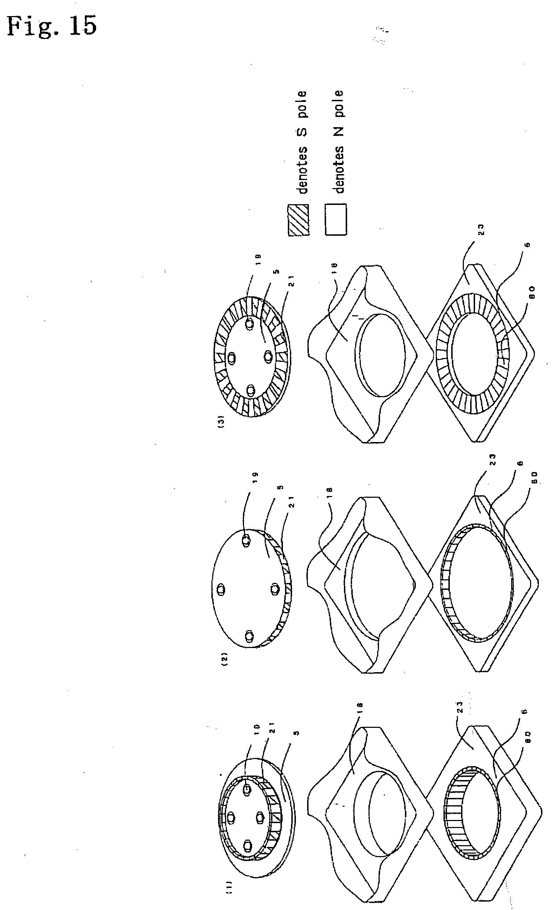

Next, an example of the sample table 5, which is driven without the motor 24, will be explained with reference to Fig. 15. In an example of Fig. 15(1), the sample table 5 has a cylinder on the bottom, and the driven magnets 21 (permanent magnet) each having different magnetic poles arranged on the peripheral wall of the cylinder by turns. In an example of Fig. 15(2), the sample table 5 is disk-like, and has the driven magnets 21 each having different magnetic poles arranged on the peripheral wall of the disk by turns. On the other hand, in the side of the sample table driving means 6, a plurality of electromagnets 80 each serving as a driving magnet are arranged on the inside of the peripheral wall of the housing unit 23 so as to be opposite to the driven magnets 21. In an example of Fig. 15(3), the driven magnets 21 are arranged circumferentially on the plane in a vertical direction. On the other hand, in the side of the sample table driving means 6, the electromagnets 80 are arranged circumferentially on the plane so as to be opposite to the driven magnets 21. The sample table driving means 6 has the electromagnets 80 arranged on the inside of the peripheral wall or the electromagnets 80 arranged circumferentially on the plane mounted on the housing unit 23. In addition, the sample table driving means 6 is attached freely-removably to the temperature-controlled chamber 15 as one body by fitting the housing unit 23 to the base board 18 with the screws. In the examples of Figs. 15(1), 15(2), 15(3), the housing unit 23 is attached removably to the bottom of the base board 18, and the inside of the temperature-controlled chamber 15 having the driven magnets 21 and the outside having the electromagnets 80 are separated by the inner wall 16. In these examples, the housing unit 23 has a function for controlling the relative positional relationship of the driving magnets, which is provided to the magnet housing 27 in the example of Fig. 2.

In all examples of Figs. 15(1), (2), (3), the electromagnets 80, which are adjacent or located at intervals of the fixed number of magnets, are arranged in the housing unit 23 so as to respectively have opposite magnetic polarities. The sample table 5 is operated by shifting magnetic field which is generated by switching the magnetic polarities of the electromagnets 80 in order through a control unit (not-illustrated) inside or outside the housing unit 23. Besides, there should be the driven magnets 21 which enough magnetic force is got to operate the sample table 5 even if they are not arranged for all circumference of the sample table 5. In addition, as the explanation about Fig. 14(2), in switching the magnetic field of the electromagnets 80, the sample table 5 is surely moved by respectively arranging the electromagnets 80 of the driving side and the magnets 19 of the driven side to the positions having balanced magnetic force at equal intervals. The sample table 5 can be smoothly operated and accurately positioned by increasing the number of the-electromagnets 80 and making it small and by sizing the driven magnet 21 equal to the electromagnet.

In these examples, rotary magnetic field is generated as shifting magnetic field. However, in moving the sample table 5 linearly, liner shifting magnetic field is generated by arranging the electromagnets linearly.

As explained in the above, the following embodiments can be carried out in this invention.
(1) In a constant-temperature equipment comprising a temperature-controlled chamber, means for controlling environment conditions in the temperature-controlled chamber, a sample table freely removably arranged in the temperature-controlled chamber, a sample shelf freely removably attached on the sample table, sample table drive means for driving the sample table from the outside of the temperature-controlled chamber, a large opening portion provided to one plane of the temperature-controlled chamber, a small opening portion provided to one side plane of the temperature-controlled chamber so that at least one sample container passes through, conveyance means for carrying in and out at least one container through the small opening portion, and a control assembly for controlling the conveyance means and the sample table drive means, the sample table drive means is freely removable and insertable outside the temperature-controlled chamber. Here, the sample shelf is to contain a plurality of containers accommodating the samples. The large opening portion is provided with a freely-opening-and-closing door to carry in and out the sample table and/or the sample shelf. The small opening portion is provided with a shielding plate which freely opens and closes.
(2) The conveyance means can be attached removably to the external wall of the temperature-controlled chamber.
(3) The sample table has a rolling body or a low frictional member on a plane opposite to the sample table drive means.
(4) The sample table drive means has magnet generating means which is operated by driving source. The sample table has the driven magnets arranged at the positions corresponding to the magnet generating means through members for forming the temperature-controlled chamber. According to this, the driving force from the driving source is transmitted to the sample table by non-contact coupling based on the magnetic force passing through the members forming the temperature-controlled chamber.
(5) A permanent magnet is used for a driven magnet which is provided to the sample table, and also for magnet generating means which is provided to the sample table drive means.
(6) A permanent magnet is used for a driven magnet which is provided to the sample table, and a direct current electromagnet is used for magnet generating means which is provided to the sample table drive means.
(7) The sample table drive means, which has no movable portions, has alternate-current electromagnets or direct current electromagnets fixed. The sample table has the driven magnets arranged so as to be opposite to the alternate current electromagnets or the direct current electromagnets through the members forming the inside of the temperature-controlled chamber. The driving force, which is generated by a variation of phases of the alternate current electromagnets or the direct current electromagnets which are adhered on the back side of the member forming the inside of the temperature-controlled chamber, is transmitted to the sample table by the non-contact coupling based on the magnetic force passing thorough the walls of the temperature-controlled chamber.
(8) The sample table drive means is attached removably on the back side of the temperature-controlled chamber. The sample table drive means has the magnet generating means which is operated by the driving source. The sample table has the driven magnets arranged so as to be opposite to the magnet generating means through the member forming the temperature-controlled chamber. According to this, the driving force from the driving source is transmitted to the sample table by the non-contact coupling based on the magnetic force passing through the member forming the temperature-controlled chamber.
(9) The conveyance means comprises a finger, an arm mechanism for moving the finger forward or rearward, slewing means for slewing the finger and the arm mechanisms, and travel means for moving the swivel means vertically or laterally.
(10) The conveyance means accommodates at least the finger and the container, and has a pass box in a route for carrying the container.
(11) In addition, the shielding plate opening and closing means is provided.
(12) The shielding plates more than one are arranged parallel to the travel means. The conveyance means is provided with engaging means for the shielding plates to open and close them by moving workings of the travel means.
(13) The temperature-controlled chamber has a lengthwise opening on one side plane. A plurality of shielding plates is vertically pilled up inside two slide-guides which are provided on the external wall of the constant-temperature equipment outside the lengthwise opening. The conveyance means lifts all the upper shielding plates including the opposite ones by engaging with the opposite ones. According to this, a small opening for passing at least one sample container is formed.
(14) The shielding plates are respectively opposite to the shelves of the sample shelf at the time when the small opening is closed.
(15) The shielding plates have either the engaging hole or the bracket engaged with the engaging means of the conveyance means.
   According to the above-mentioned construction, the constant-temperature equipment can rotate the sample table by having a turntable on the sample table drive means and arranging either one kind of the permanent magnets or the direct current electromagnets circularly for the center on the turntable.
(16) The constant-temperature equipment comprises a temperature-controlled chamber, means for controlling environment conditions in the temperature-controlled chamber, a sample table freely removably arranged in the temperature-controlled chamber, a sample shelf freely removably attached on the sample table, sample table drive means for driving the sample table from the outside of the temperature-controlled chamber, a large opening portion provided to one plane of the temperature-controlled chamber, a small opening portion provided to one side plane of the temperature-controlled chamber so that at least one sample container passes through, conveyance means for bring in and out at least one container through the small opening portion, and a control assembly for controlling the conveyance means and the sample table drive means. Here, the sample shelf is to contain a plurality of containers accommodating the samples. The large opening portion is provided with a freely-opening-and-closing door to carry in and out the sample table and/or the sample shelf. The small opening portion is provided with a shielding plate which freely opens and closes. In addition, the conveyance means is outside the temperature-controlled chamber, being attached removably on the external wall thereof, besides having means for transmitting the driving force to the sample table by the non-contact coupling based on the magnetic force which passes through the walls of the temperature-controlled chamber for separating from the outside.
(17) The temperature-controlled chamber is a chamber for accommodating the sample, being a part of the constant-temperature equipment having the function for controlling the density of gas such as humidity, oxygen, nitrogen and carbon dioxide besides at least keeping a fixed indoor temperature. Specially, the constant-temperature equipment of this invention is most suitable for a culture tank which is used in biological field.
(18) The non-contact coupling based on the magnetic force can be carried out by combinations more than one among the permanent magnets and the permanent magnets, the permanent magnets and the direct current electromagnets, and the permanent magnets and the alternate current electromagnets. In addition, stronger magnetic force can be obtained by eliminating the dispersion of flux by respectively locating yokes to the magnets.
(19) The sample table drive means has magnetic housing which rotates or linearly moves, having either one kind of the permanent magnets or the direct current electromagnets arranged on the magnetic housing as the magnet generating means. The sample table has the permanent magnets arranged as the driven magnet. Movement of the magnetic housing from the driving source of the sample table drive means is turned into following movement of the sample table by the non-contact coupling based on the magnetic force through the members forming the inside of the temperature-controlled chamber.
(20) The direct current electromagnets or the alternate current electromagnets can be used for the magnet generating means arranged on the magnetic housing. The strength of magnetic force can be controlled by using the electromagnets, thereby easily enabling to seek the magnetic force most suitable for transmitting the drive force to the sample table.
(21) The sample table can be worked without using the rotary drive force from the motor. In using the direct current electromagnets, the direct current electromagnets are fixed on the direct current electromagnets instead of the permanent magnets, and the magnetic poles of the direct current electromagnets are switched by the control assembly in order. In this way, the sample table can be followed by step motion. In this case, the sample table can be precisely positioned by raising the density within the fixed interval of the direct current electromagnets.
(22) The alternate current electromagnets can be also used instead of the direct current electromagnets. In this case, it can be made a direct drive motor type, which drives the permanent magnets located on the back side of the sample table as a movement terminal. Even if any driving method is adopted, the sample table drive means can be repaired and maintained without influencing an environment in the temperature-controlled chamber even during testing or culturing by making the structure to be attached removably. Since there are heat insulating materials between the inside of the temperature-controlled chamber and the installation part of the sample table drive means, it is possible to evade that the structure components of the sample table drive means are influenced by the atmosphere such as high temperature and high humidity in the temperature-controlled chamber during testing or culturing.
(23) The sample table is put in the temperature-controlled chamber so as to be movable. The movement method may be rotary motion or linear motion to right and left for the small opening. Preferably, it is rotated around a perpendicular line which passes through the center of the temperature-controlled chamber.
(24) The sample table has rolling means or members having low frictional residence used as base material provided on the back side to surely follow the movement of the sample table drive means. It is desirable that the permanent magnets located on the back side of the sample table are separated by a constant distance from the floor of the temperature-controlled chamber or covered with the members having low frictional resistance so as not to be rubbed. Similarly, in case the sample table drive means has the permanent magnets or the direct current electromagnets, it is desirable to get rid of friction load by separating from the back side of the floor of the temperature-controlled chamber by a constant distance because these magnets move in themselves.
(25) The sample shelf is so constructed that the sample containers are vertically put on a plurality of shelves. The shelves may be laterally fixed in a plurality of rows, or may be separated by every vertical one line. In case of a disk-like sample table, the shelves are constructed so as to be integrated in a cylindrical shape along the lengthwise and the circumference or so as to be separated by every vertical line. In the latter case, the disk-like sample table has portions for carrying in and out arranged in a circle for the outside. Although the shelves are put on the top or the side of the positioning tool provided on the sample table, they can be fixed with screws or springs so as not to cause misregistration.
(26) Since the large opening formed on one side of the temperature-controlled chamber is hardly opened and closed while the temperature-controlled chamber is driven, it is opened and closed with a door by hand control. Here, the door may be a hinge type or a male-female type, and mounted on the side or the ceiling. The door is preferably provided to the side of the temperature-controlled chamber, and may have a glass window fitted to observe the inside.
(27) The small opening is preferably provided just before an optional shelf not a uniformity place of the side of the temperature-controlled chamber because it is opened at the positions of a plurality of shelves. The small opening has the opening area enough size that the conveyance means can carry in and out at least one sample container. The shielding plate of the small opening may be made in a well-known shape or type. However, generally, in case of the door having hinges at the upper and the lower, there is a risk of disturbing of the atmosphere in the temperature-controlled chamber by causing an air current according to working opening-and-closing door and by aggregating the atmosphere in the temperature-controlled chamber with the atmosphere of the outside.
   Therefore, there is no disorder of the air current according to the working for opening-and-closing by using shielding plates, which open and close with sliding vertically or laterally, as a type for opening-and-closing door. The shielding plates are arranged between the conveyance means and the external wall of the temperature-controlled chamber in parallel to the travel means without a gap. Here, the number of shielding plates is the same as the shelves.
(28) The sample shelf has the shelves arranged at an equal interval in the height and the shielding plates may be corresponded to the shelves. For example, in putting a sample container having the size for two shelves on the sample shelf, a partition between the shelves is removed. The size of the shielding plate may be equal to the height of each shelf of the sample shelf or may not. However, when it is recognized that a big container is put on beforehand, the pass box or the dome must be the size enough to contain the big container.
(29) The conveyance means comprises a finger, arm mechanism for advancing and backing the finger, and travel means for laterally or vertically moving the finger and the arm mechanism. The conveyance means has a pass box having the minimum capacity necessary for carrying the sample container, being constructed so as not to have a harmful influence on the internal environment in carrying the sample container in and out. Although it is preferable that there is no opening between the pass box and the shielding plates, means for improving the tightness such as a packing etc. also can be provided.
(30) The conveyance means is attached removably on the external wall adjacent to the small opening of the temperature-controlled chamber, thereby enabling to repair and maintain without affecting the environment in the temperature-controlled chamber even during testing or cultivating. Since the conveyance means is mounted on the outside, the components of conveyance means are not affected by the atmosphere of high temperature and high humidity in the temperature-controlled chamber.
(31) The conveyance means has means for engaging with the shielding plates, opening-and-closing the shielding plates from the outside of the temperature-controlled chamber without a mechanism for opening-and-closing them. That is, the conveyance means and the shielding plates are integrally interlocked by the engaging means, enabling to open-and-close the shielding plates by the travel means of a part of the conveyance means without a mechanism exclusive for opening-and-closing. According to this, the conveyance means has few components and simple structure, thereby enabling reduction in cost and improvement in reliability. As for the engaging means, mechanical connection due to bar members or connection due to magnetic force is considered.
(32) The shielding plates can be lined up by the slide-guides movably in the vertical direction or the lateral direction. The shielding plates can improve the tightness between the adjoining portions by forming the sections of the adjoining portions in curb or a plurality of straight lines which can be interfitted.
(33) To improve the tightness between the shielding plate and the external wall of the constant-temperature equipment, the shielding plate is made of a member which is attracted by the magnetic force or provided with the member and besides, a magnet is provided at the position where contacts to the shielding plate on the external wall of the temperature-controlled chamber. According to this, the shielding plate and the external wall of the temperature-controlled chamber are adhered by the magnetic force, thereby enabling to improve the tightness.
(34) Pulse motors such as a stepping motor and a servomotor are preferably used as a motor for the conveyance means or the sample table drive means. A control device is provided to automatically control these motors and to control the environmental conditions in the temperature-controlled chamber.
(35) It is preferable that there are no projections such as a rotating axis and a fixed shelf in the temperature-controlled chamber of the constant-temperature equipment. Further, it is preferable that the inside of the temperature-controlled chamber is smooth so as to be easily cleaned. However, it is possible to form holes for supplying or discharging gasses such as humidity air for controlling the environmental conditions, oxygen, nitrogen and carbon dioxide, to provide with sensors for the temperature, the humidity and the concentration of various gasses. In this case, the inside may be constructed so as not to be influenced by the outside environment by making it positive pressure.
(36) The well-known technology such as a sump for humidification and a mechanism for de-dew-condensation, may be used in the temperature-controlled chamber. The temperature-controlled chamber can pool demineralized water because the floor is flat without mechanical components. In addition, the humidity during testing and culturing can be maintained by forming the sump out of concaves composed of smooth curved plane or by placing vats at four corners of the floor of the temperature- controlled chamber.
(37) As a detachable pierce, magnetic coupling for absorbing the shielding plate with the electromagnets can be used instead of mechanical coupling such as the engaging pin.
(38) In Fig. 2, the sample table drive means 6 is so constructed that all, components of the gear motor 24, the pulleys 25a, 25b, the belt 26 and the magnetic housing 27 are mounted on the housing unit 23. However, at least, the magnetic housing 27 should be rotatitively mounted thereon. The magnetic housing 27 can be installed on the bottom of the temperature-controlled chamber 15 by installing the housing unit 23 on the base board 18.

According to these embodiments, the following effects can be obtained.

As shown in Fig. 2, because all of electrical and mechanical structures such as the sample shelf 4, the sample table 5, the sample table drive means 6, the conveyance means 11 and the travel means 12 are removed from the temperature-controlled chamber 15, dry sterilization at a high temperature, which was difficult with the conventional automatic incubators, can be carried out all of the structures in the temperature-controlled chamber 15. In this case, the components do not break down at the high temperature at the time of the dry sterilization by removing the electrical and mechanical structures.

Since the temperature-controlled chamber has no irregularities due to the electrical and mechanical structures, it is possible to easily carry out wiping before and after the sterilization and carry out highly reliable sterilization. Therefore, it is possible to carry out highly reliable culturing and testing. Since the sample shelf 4 and the sample table 5 can be easily removed from the temperature-controlled chamber 15, they can be individually sterilized and washed in sterilized liquid.

Since the temperature-controlled chamber 15 has no electrical and mechanical structures for the driving portion and has the insulating materials 22 put along the portion connected to the sample table drive means 6, the driving portion is not affected by the atmosphere in the high temperature and humidity. Therefore, the driving portion hardly breaks down, thereby enabling to avoid from suspensions or interruptions of the culturing and testing caused by the trouble.

If the electrical and mechanical structures should break down during the culturing and testing, the sample table drive means 6 or the conveyance means 11 can be removed and repaired because the sample table drive means 6, the conveyance means 11 and the temperature-controlled chamber 15 are completely separated one another. Therefore, it is possible to avoid from the suspensions of the culturing and testing. In addition, good culture environment can be maintained by providing with means for keeping humidity during the culturing and testing.

Since the temperature-controlled chamber 15 has the plane bottom without mechanical components, it is possible to pool pure water therein to maintain the humidity during the culturing and testing.

Since the conveyance means 11 has the pass box having the minimum capacity necessary for carrying the container, the internal environment, which is kept in constant temperature and humidity, is not affected in carrying in and out the container. Therefore, it is possible to maintain stable culturing and testing environment.

Since the slide type is adopted for opening and closing the shielding plates and ones except the required openings are always closed, unnecessary air-flow is not generated in the temperature-controlled chamber at the time of the opening and closing. Therefore, it is possible to maintain stably culturing and testing environment.

## Claims

1. Constant-temperature equipment comprising:
a temperature-controlled chamber (15) having a closed space surrounded by walls;
a sample table (5) arranged removably in the temperature-controlled chamber (15), for placing a sample shelf (4) for putting containers containing samples;
a plurality of driven magnets (21) installed on the sample table (5);
a plurality of driving magnets (30) arranged correspondingly to positions of the sample table (5) having the driven magnets (21) installed;
means for installing the driving magnets (30) from an outside through a wall of the temperature-controlled chamber (15), said driving magnets (30) being magnetically combined with the driven magnets (21); and
means for generating shifting magnetic field according to the driving magnets (30).

2. Constant-temperature equipment according to claim 1, wherein the means for installing is a housing unit (23) for having the driving magnets (30) mounted and for prescribing mutual configuration of the driving magnets (30).

3. Constant-temperature equipment according to claim 1, wherein the sample table (5) has a rolling body or low frictional resistance member on a plane opposite to sample table drive means (6).

4. Constant-temperature equipment according to claim 1, wherein the means for installing comprises a magnetic housing (27) for having the driving magnets (30) mounted and for prescribing mutual configuration of the driving magnets (30), and a housing unit (23) combining the magnetic housing (27) rotatitively.

5. Constant-temperature equipment according to claim 1, wherein the driving magnets (30) are permanent magnets and the means for generating shifting magnetic field comprises permanent magnets.

6. Constant-temperature equipment according to claim 1, wherein the driving magnets (30) are permanent magnets and the means for generating shifting magnetic comprises electromagnets.

7. Constant-temperature equipment according to claim 6 wherein the shifting magnetic field is generated by switching magnetic poles of the electromagnets.

8. Constant-temperature equipment according to any preceding claim, wherein the driven magnets (21) are circularly arranged to a center of the sample table, and the sample table (5) is rotated by circularly arranging the driven magnets (21).

## Patentansprüche

1. Konstanttemperatur-Vorrichtung mit:
einer temperaturgesteuerten Kammer (15), die einen geschlossenen Raum aufweist, der von Wänden umgeben ist;
einem Probentisch (5), der bewegbar in der temperaturgesteuerten Kammer (15) angeordnet ist zur Platzierung eines Probenregals (4) für Container, die Proben aufweisen;
einer Mehrzahl von angetriebenen Magneten (21), die auf dem Probentisch (5) installiert sind;
einer Mehrzahl von antreibenden Magneten (30), die entsprechend den Positionen des Probentisches (5) angeordnet sind, der die installierten angetriebenen Magnete (21) aufweist;
einem Mittel zum Installieren der antreibenden Magnete (30) von außen durch eine Wand der temperaturgesteuerten Kammer (15), wobei die antreibenden Magnete (30) magnetisch mit den angetriebenen Magneten (21) gekoppelt sind; und
einem Mittel zum Erzeugen einer Magnetfeldverschiebung gemäß den antreibenden Magneten (30).

2. Konstanttemperatur-Vorrichtung nach Anspruch 1, bei der das Mittel zum Installieren eine Gehäuseeinheit (23) ist zur Montage der antreibenden Magnete (30) und zum Festlegen einer wechselseitigen Konfiguration der antreibenden Magnete (30).

3. Konstanttemperatur-Vorrichtung nach Anspruch 1, bei der der Probentisch (5) einen Rollkörper oder ein Bauteil mit geringem Reibwiderstand auf einer Ebene aufweist, die einem Tischantriebsmittel (6) gegenüberliegt.

4. Konstanttemperatur-Vorrichtung nach Anspruch 1, bei der das Mittel zum Installieren ein Magnetgehäuse (27) aufweist zur Montage der antreibenden Magnete (30) und zur Festlegung einer wechselseitigen Konfiguration der antreibenden Magnete (30), und eine Gehäuseeinheit (23), die das Magnetgehäuse (27) drehbar koppelt.

5. Konstanttemperatur-Vorrichtung nach Anspruch 1, bei der die antreibenden Magnete (30) Dauermagnete sind, und das Mittel zum Erzeugen der Magnetfeldverschiebung Dauermagnete aufweist.

6. Konstanttemperatur-Vorrichtung nach Anspruch 1, bei der die antreibenden Magnete (30) Dauermagnete sind, und das Mittel zum Erzeugen der Magnetfeldverschiebung Elektromagnete aufweist.

7. Konstanttemperatur-Vorrichtung nach Anspruch 6, bei der die Magnetfeldverschiebung durch Schalten von Magnetpolen der Elektromagnete erzeugt wird.

8. Konstanttemperatur-Vorrichtung nach einem der vorangegangenen Ansprüche, bei der die angetriebenen Magnete (21) kreisförmig um ein Zentrum des Probentisches angeordnet sind, und der Probentisch (5) durch kreisförmiges Anordnen der angetriebenen Magnete (21) gedreht wird.

## Revendications

1. Equipement à température constante, comprenant:
une chambre à température contrôlée (15) présentant un espace fermé entouré par des parois;
une table à échantillons (5) agencée de façon amovible dans la chambre à température contrôlée (15), pour y placer une étagère à échantillons (4) destinée au dépôt de récipients contenant des échantillons ;
une multiplicité d'aimants entraînés (21) installés sur la table à échantillons (5);
une multiplicité d'aimants d'entraînement (30) agencés de façon correspondante à des positions de la table à échantillons (5) où les aimants entraînés (21) sont installés;
des moyens pour installer les aimants d'entraînement (30) à partir d'un côté extérieur à travers une paroi de la chambre à température contrôlée (15), lesdits aimants d'entraînement (30) étant magnétiquement combinés avec les aimants entraînés (21); et
des moyens pour produire un champ magnétique de déplacement selon les aimants d'entraînement (30).

2. Equipement à température constante selon la revendication 1, dans lequel les moyens d'installation sont une unité de boîtier (23) destinée à avoir les aimants d'entraînement (30) montés et à prescrire une configuration mutuelle des aimants d'entraînement (30).

3. Equipement à température constante selon la revendication 1, dans lequel la table à échantillons (5) comporte un corps roulant ou un élément à faible résistance de frottement sur un plan opposé aux moyens d'entraînement (6) de la table à échantillons.

4. Equipement à température constante selon la revendication 1, dans lequel les moyens d'installation comprennent un boîtier magnétique (27) destiné à avoir les aimants d'entraînement (30) montés et à prescrire une configuration mutuelle des aimants d'entraînement (30), et une unité de boîtier (23) combinant le boîtier magnétique (27) en rotation.

5. Equipement à température constante selon la revendication 1, dans lequel les aimants d'entraînement (30) sont des aimants permanents et les moyens pour produire un champ magnétique de déplacement comprennent des aimants permanents.

6. Equipement à température constante selon la revendication 1, dans lequel les aimants d'entraînement (30) sont des aimants permanents et les moyens pour produire un champ magnétique de déplacement comprennent des électroaimants.

7. Equipement à température constante selon la revendication 6, dans lequel le champ magnétique de déplacement est produit en commutant des pôles magnétiques des électroaimants.

8. Equipement à température constante selon l'une quelconque des revendications précédentes, dans lequel les aimants entraînés (21) sont disposés en cercle par rapport à un centre de la table à échantillons, et la table à échantillons (5) est mise en rotation en agençant en cercle les aimants entraînés (21).
